Europäisches Patentamt

European Patent Office

Office européen des brevets

(11) Publication number: **0 418 960 A2**

# EUROPEAN PATENT APPLICATION

(21) Application number: **90202440.5**

(51) Int. Cl.5: **C12Q 1/68**

(22) Date of filing: **14.09.90**

(30) Priority: **22.09.89 US 411443**

(43) Date of publication of application:
**27.03.91 Bulletin 91/13**

(84) Designated Contracting States:
**AT BE CH DE DK FR GB IT LI LU NL SE**

(71) Applicant: **EASTMAN KODAK COMPANY**
**343 State Street**
**Rochester, New York 14650(US)**

(72) Inventor: **Wu, Annie Liu, c/o Eastman Kodak**
**Company**
**343 State Street, Rochester,**
**New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) Nucleic acid detection method using unequal primer concentrations in polymerase chain reaction.

(57) The detection of nucleic acids can be achieved with certain advantages in polymerase chain reactions by using primers in unequal concentrations. In particular, the molar ratio of a pair of primers is at least 2:1. Amplification of the nucleic acid of interest is carried out using standard reagents, but denaturation of the complementary products prior to detection is not needed. Detection is then accomplished using an enzyme-labeled reagent which will provide a colorimetric or fluorometric signal in the presence of the appropriate substrate.

EP 0 418 960 A2

# NUCLEIC ACID DETECTION METHOD USING UNEQUAL PRIMER CONCENTRATIONS IN POLYMERASE CHAIN REACTION

The present invention is directed to a method for detecting a nucleic acid in polymerase chain reactions. It can be used to detect nucleic acids in diagnostic assays or genetic research where nucleic acids associated with genomic, bacterial or viral DNA are of interest.

Nucleic acid probe technology has developed rapidly in recent years as researchers have discovered its value for detection of various diseases, organisms or genetic features which are present in very small quantities in a test sample. The use of probes is based upon the concept of complementarity. In DNA the two strands are bound to each other by hydrogen bonds between complementary nucleotides (also known as nucleotide pairs).

The DNA complex is normally stable, but the strands can be separated (or denatured) by conditions which disrupt the hydrogen bonding. The released single strands will reassociate only with another strand having a complementary sequence of nucleotides. This hybridization process can occur in solution or on a solid substrate.

A target nucleic acid sequence of DNA of a target organism or cell may be only a small portion of the total strand, so that it is very difficult to detect its presence using most known labeled DNA probes. Much research has been carried out to overcome this problem including improvements in probe sensitivity and synthesis of nucleic acids.

A significant advance in the art is described in US-A-4,683,195 and US-A-4,683,202. Without going into extensive detail, these patents describe an amplification method wherein primers are hybridized to nucleic acid templates in the presence of a polymerization agent (such as a polymerase) and deoxyribonucleoside triphosphates, and extension products are formed from the primers. These products are denatured and used as templates in a cycling reaction which amplifies the number and amount of existing nucleic acids to facilitate their subsequent detection. The amplification method can be carried out cyclically as many times as desired to produce a larger quantity of detectable material from a small amount of target nucleic acid sequence.

A more recent development for DNA sequencing is described by Gyllensten et al ( Proc.Natl.Acad.Sci. USA , 85 , pp. 7652-7656, October, 1988). Single-stranded DNA is generated in polymerase chain reaction procedures using a limiting amount of one of the primers in a primer pair. Such DNA can be used for direct sequencing of a gene, such as the HLA-DQα locus, and for generation of hybridization probes. Detection of the nucleic acid fragments in the sequencing is achieved by use of radioisotope-labeled deoxyribonucleoside triphosphates. Radioisotope-labeled probes are similarly prepared.

This art fails, however, to consider the use of unequal primer concentrations in polymerase chain reaction detection methods or the advantages it may provide in certain assays.

While the use of radioisotopes as labels for detecting nucleic acids is known and may provide certain advantages, it has considerable drawbacks in the preparation of convenient, safe and simple methods and diagnostic test kits having long shelf life. The hazards are handling problems from using radioisotopes are apparent. One would not readily package them in a test kit for use in doctor's offices or remote sites. Moreover, many isotopes commonly used in nucleic acid probes have a short (that is, a few days) half life. Thus, they have limited shelf life.

It would be advantageous to avoid the problems presented with the use of radioisotopes. Such a means might be provided with enzyme labels which are commonly used in various immunological assays. However, many enzymes, including peroxidase are heat sensitive and are deactivated at high temperatures. Polymerase chain reaction procedures often require high temperature conditions for certain steps, including denaturation of the primer extension products prior to detection. There is a likelihood that enzyme labels can be deactivated during denaturation if care is not taken in the process to avoid it. Thus, there is a continued need for a way to provide a safe, convenient and simple method of detecting nucleic acid with a diagnostic test kit having acceptable shelf life.

The problems of known procedures have been overcome using a method for the detection of a nucleic acid having two complementary strands which comprises:

A. contacting the denatured complementary strands of a nucleic acid with:

deoxyribonucleoside-5′-triphosphates,

an agent for polymerization, and

a pair of primers which are substantially complementary to the denatured nucleic acid strands

under hybridizing conditions so as to form hybridized extension products of the primers and their complementary nucleic acid strands, which extension products, when separated from their complements, can serve as templates for synthesis of the extension products,

the primers being present in a molar ratio of at least 2:1,

B. separating the primer extension products from the templates on which they were synthesized,

C. subjecting the separated primer extension products to amplifying conditions, and

D. without further denaturation of the amplified products formed in step C, colorimetrically or fluorometrically detecting the presence of the primer extension product which was formed using the primer in excess, the detection accomplished using an enzyme-labeled reagent, which in the presence of its substrate, will provide a detectable colorimetric or fluorometric signal, the reagent being either complementary to the detected primer extension product, or capable of being associated therewith.

The present invention provides several advantages and thus, an advance in the art for detecting nucleic acids. It is a method which is convenient, simple to carry out, safe and sensitive to low concentrations of targeted nucleic acid. It can be carried out using a simple diagnostic test kit having acceptable shelf life and which would not present unnecessary hazards in a doctor's office or other testing sites.

These advantages are achieved by using enzyme detection reagents which can be used to provide a colorimetric or fluorometric signal to indicate the presence of the targeted nucleic acid. Radioisotopes are avoided along with their disadvantages. Moreover, the enzyme detection reagents are used in combination with an unequal amount of primers in the amplification procedures so that denaturation prior to detection of the product from the excess primer can be avoided. Heat-sensitive enzyme labels are thereby protected from deactivation.

The present invention is directed to the detection of one or more specific nucleic acid sequences present in one or more predetermined (that is, targeted) nucleic acids in a test specimen. Such samples can include cellular or viral material, hair, body fluids, or other materials containing bacterial, viral or genomic DNA or RNA which can be detected.

The invention is especially useful when combined with a chain reaction for producing, in exponential quantities relative to the number of reaction steps involved, at least one predetermined nucleic acid sequence. The product will be a discrete nucleic acid duplex with termini corresponding to the ends of the specific primers employed. Any source of nucleic acid, purified or not, can be utilized as the starting material provided it contains or is suspected of containing the specific nucleic acid targeted for detection. A mixture of nucleic acids can be employed if desired. The sequence to be duplicated can be a fragment or the entire nucleic acid. Moreover, more than one nucleic acid sequence can be detected simultaneously by using a set (or pair) of primers for each sequence to be detected. The sequences can be in the same or different nucleic acids.

Nucleic acids can be obtained from various sources including plasmids or naturally occurring DNA from any plant, animal or human source. It may be extracted from various tissues including blood, peripheral blood mononuclear cells, tissues or other sources known in the art. This invention is particularly useful for the detection of Human Leukocyte Antigen DNA or viral DNA, such as from human papilloma viruses, RNA viruses and retroviruses (for example, HIV-I), extracted from fluids and cells infected by the virus where heterogeneity within and among infected individuals is prevalent.

DNA is isolated from the cells in a specimen using any suitable technique, many of which are well known in the art, as long as the technique does not degrade the DNA. For example, it can be extracted from cells using a buffered protease or proteinase K composition at a temperature and pH which reduces the likelihood that the DNA will be degraded, as described, for example by Kan et al, New Eng.J.Med. , 297 , pp. 1080-1084 (1977) and in Nature , 251 , pp. pp. 392-393 (1974). Other extraction techniques are described by Maniatis et al ( Molecular Cloning, A Laboratory Manual , Cold Spring Harbor Laboratory, 1982) and in EP-A-0 145 356, EP-A-0 240 191, and EP-A-0 245 945, and by Nunberg et al, Proc. Nat. Acad. Sci.(USA) , 75 (11), pp. 5553-5556, 1978 and Saiki et al, Bio/Technology , 3 , pp. 1008-1012 (1985).

The extracted DNA can be purified by dialysis, chromatography, affinity capture using complementary nucleic acids or by any other suitable technique readily apparent to one skilled in the art.

Once isolated DNA is obtained, it is subjected to an amplification procedure which produces exponential quantities of the molecule relative to the number of steps involved. It is to be understood that where several DNA molecules are of interest, the amplification process can be used to multiply all of them. The end result is a large number of detectable nucleic acids which can then be subjected to procedures for detection.

The amplification method generally used is described in considerable detail in US-A-4,683,195 and US-A-4,683,202.

As used herein in referring to primers or probes, the term "oligonucleotide" refers to a molecule comprised of two or more deoxyribonucleotides or ribonucleotides, and preferably more than three. The exact size is not critical (except for the probe described below) but depends upon many factors including

3

the ultimate use or function of the oligonucleotide. The oligonucleotide may be derived synthetically or by other methods known in the art.

The term "primer" refers to an oligonucleotide, whether naturally occurring or synthetically produced, which is capable of acting as a point of initiation of synthesis when placed under conditions in which synthesis of a primer extension product complementary to a nucleic acid strand is induced. Such conditions include the presence of nucleotides (such as the four standard deoxyribonucleotide triphosphates) and an agent for polymerization such as a DNA polymerase, suitable temperature and pH, and other suitable reagents such as a source of magnesium or manganese ions. Primers are generally completely complementary to the nucleic acid sequence of interest, but some mismatches between target and primer may exist which do not significantly adversely affect priming and formation of primer extension product. This is what is meant by "substantially complementary".

A pair of primers can be used in the amplification according to this invention specific for a particular nucleic acid. In each pair of primers, it is critical that the molar concentrations of the primer be unequal, generally in a molar ratio greater than 2:1. Preferably, the molar ratio is at least 5:1 and can be a greater ratio if desired. The primer present in the greater amount for each primer pair is considered the primer present in excess. The appropriate molar ratio for a given targeted nucleic acid would depend upon the amount of target nucleic acid, the number of amplification cycles used and the sensitivity of the detection reagents, and would be readily determined by one skilled in the art. It is important that the ratio be established for effective amplification in the polymerase chain reaction, and effective detection of one primer extension product without the need for denaturation.

The amount of primer present in lesser amount in a given primer pair would also be readily determined by one skilled in the art from known literature and modest experimentation. Generally, it is present in an amount of at least 0.001 $\mu$molar, with from 0.005 to 0.5 $\mu$molar being preferred. Generally, the amount is effective to provide sufficient amplification of the targeted strand.

Primers are generally single-stranded. The exact length of each primer will vary depending upon the use contemplated, the complexity of the target sequence, reaction temperature and the source of the primer. Generally, the primers used in this invention will have from 15 to 50 nucleotides, and preferably, they have from 20 to 30 nucleotides.

Primers useful herein can be obtained from a number of sources or prepared using known techniques and equipment, including for example, an ABI DNA Synthesizer (available from Applied Biosystems) or a Biosearch™ 8600 Series, 8700 Series or 8800 Series Synthesizer (available from Milligen-Biosearch, Inc.) and known methods for their use. Naturally occurring primers isolated from biological sources are also useful (such as restriction endonuclease digests).

Once the targeted DNA has been extracted (if necessary) and denatured, its strands are contacted with the pair of primers described herein under conditions such that a mixture of hybridized products of primers and target DNA strands are formed. Such conditions are those normally used for amplification as described in US-A-4,683,202, with the exception of the unequal amounts of the primers. Primer extension products are then formed with at least one of the hybridized products followed by additional priming and extension product formation. After the last cycle of amplification, the product formed with the primer in excess is detected in a suitable manner without the need for a final heating, or denaturation, step.

The present invention is also useful for the detection of a specific nucleic acid having two complementary strands. Most nucleic acid sequences of interest already are double-stranded, such as those found in DNA. However, single-stranded nucleic acid sequences, such as mRNA, can be similarly detected after they are converted to a double-stranded sequence using reverse transcriptase.

A predetermined nucleic acid sequence is reproduced using a nucleic acid containing that sequence as a template. If the nucleic acid contains two strands, it is necessary to separate the strands, either as a separate step (if the polymerase is not thermostable) or simultaneously with the formation of primer extension products. If needed, denaturing can be accomplished using any suitable physical, chemical or enzymatic means as described in the art, such as by heating.

Once the separated strands are available for use, synthesis of additional nucleic acid strands can be carried out using the primers in a buffered aqueous solution generally at a pH of from 7 to 9. The deoxyribonucleotide triphosphates dATP, dCTP, dGTP and dTTP are also added to the synthesis mixture in adequate amounts, and if denaturing is needed, the resulting solution is heated to 90-100°C for up to 10 minutes. After this heating, the solution is preferably cooled to room temperature, and an appropriate agent for inducing (or catalyzing) the formation of primer extension products is introduced. This inducing agent is generally known in the art as a polymerization agent. Reaction to form these products is carried out under known conditions (generally from room temperature to that temperature at which polymerization no longer occurs).

4

The polymerization agent may be any compound, or combination of reagents, which will function to accomplish the synthesis of primer extension products, including enzymes (for example, E. coli DNA polymerase I, T4 DNA polymerase, Klenow polymerase, reverse transcriptase and others known in the art). Particularly useful enzymes are thermally stable enzymes, cloned or naturally occurring, such as those obtained from various Thermus bacterial species.

Preferred thermally stable enzymes are DNA polymerases isolated from either Thermus aquaticus or Thermus thermophilus , or synthetically prepared from a genome thereof by cloning methods as described in EP-A-0 258 017 and WO-A-89/06691. Other useful enzymes are described by Rossi et al, Syst. Appl. Microbiol. 7 (2-3), pp. 337-341, 1986. Generally, the synthesis of extension products will be initiated at the 3′ end of each primer and proceed in the 5′ to 3′ direction along the template until synthesis is terminated.

The newly formed primer extension products comprising the newly synthesized strands and their respective primers form double-stranded molecules with the initial target strands which are used in the succeeding steps of the method. These strands are then separated by denaturation as described above to provide single-stranded molecules, onto which new nucleic acids are synthesized as described above. Additional reagents may be needed to keep the amplification procedure going, after which most of the extension products will consist of the specific nucleic acid sequence bounded by the primers (that is, complementary products).

The steps of strand separation and extension product synthesis can be repeated as often as needed to produce the desired quantity of the specific nucleic acid needed for the use, for example detection. Generally, the sequence of steps (commonly known as a cycle) is repeated at least once, and preferably at least 15 to 50 times. At some point in the amplification procedure, the primer present in the lesser amount will be used up, while amplification proceeds with the other primer. Thus, an excess of one primer extension product will be formed over the other product. This excess product can be detected with this invention.

When it is desired to produce more than one specific nucleic acid from the first nucleic acid or a mixture thereof, the appropriate number of pairs of primers are used in the general procedure described above.

Generally, once a desired amount of the excess primer extension product has been generated, it can be detected using an enzyme-labeled reagent which directly or indirectly provides a colorimetric or fluorometric signal in the presence of the appropriate enzyme substrate. Useful enzymes and substrates are discussed in more detail below.

Detection of the primer extension product produced from the excess primer can be accomplished using any suitable technique, including the standard dot blot procedures and equipment.

In one embodiment, the reagent is an enzyme-labeled probe which is complementary to at least one nucleic acid sequence of the primer extension product to be detected. Such probes can be prepared using procedures and chemistry known in the art, such as described for example in WO-A-0 89/02932 which is representative of art showing peroxidase-labeled probes. Such probes hybridize with the primer extension product present in excess, and the resulting hybridization product can be detected after it is separated from the non-hybridized materials by suitable means, for example, using a capture reagent, such as an insoluble reagent having avid in attached to a polymeric bead. The avidin can be used to complex with biotin moieties on the primer extension product.

In another and preferred embodiment, the enzyme-labeled reagent comprises an enzyme conjugated to a specific binding moiety. The resulting conjugate can be associated with the primer extension product of interest by complexation of the specific binding moiety with its counterpart receptor on the primer extension product present in excess. For example, an avidin-peroxidase conjugate can be used to complex with a biotinylated primer extension product present in excess. Other specific binding complexes can be likewise designed and used (such as sugar-lectin and antibody-hapten). Such materials and means for preparation are well known in the art. The resulting complex product of biotinylated primer extension product and avidin-peroxidase conjugate can be separated from uncomplexed materials using a capture probe which is complementary to the primer extension product. Capture probes are well known in the art and include polymeric particles to which oligonucleotides are suitably attached.

In the preferred embodiment described above, it is most preferred that the primer present in excess be biotinylated so that the resulting primer extension product present in excess can be complexed with an avidin-enzyme conjugate for subsequent detection. That is, the primer has a biotin moiety covalently attached thereto. Such conjugates of primer and biotin can be readily prepared using known technology, such as noted above in WO-A-0 89/02931.

If separation of hybridized products which are insolubilized is desired, any suitable separation means, including centrifugation, filtration and washing can be used. Preferably, a microporous filtration membrane is used and supplied as part of a diagnostic test kit. Particularly useful microporous filter membranes include polyamide membranes marketed by Pall Corp. If desired, the membranes may be coated with proteins, such as casein, succinylated casein or with nonionic surfactants.

The membranes can be used as a separate substrate with suitable containers for collecting fluid and soluble materials. Preferably, however, they are mounted as part of a test device. Various test devices are known in the art including those described in US-A-3,825,410, US-A-3,888,629, US-A-3,970,429 and US-A-4,446,232. Particularly useful devices are described in EP-A-0 308 231.

With contact of the enzyme-labeled reagent with the primer extension product, an appropriate substrate and any other reagents needed to produce a detectable colorimetric or fluorometric signal are provided. Useful enzyme labels include peroxidase, glucose oxidase, $\beta$-galactosidase, urease, alkaline phosphatase. Peroxidase and alkaline phosphatase are preferred with peroxidase being most preferred.

Useful substrates and dye-providing reagents are known in the art for a given enzyme. For example, useful dye-providing reagents for peroxidase include tetramethylbenzidine and derivatives thereof, and leuco dyes, such as triarylimidazole leuco dyes (as described in US-A-4,089,747). Particularly useful dye-providing compositions include triarylimidazole leuco dyes used in combination with certain stabilizing materials, for example those described in EP-A-0 308 236.

The presence of the dye is an indication of the presence of the targeted DNA in the specimen tested. The dye can often be observed visually without the use of equipment, but in some instances, the dye density may be faint or outside the visible region of the electromagnetic spectrum, and thus require suitable detection equipment (that is, spectophotometers or fluorometers) for adequate determination. Procedures for doing this are well known in the art.

The method of this invention can be used to amplify or detect nucleic acids associated with infectious diseases, genetic disorders or cellular disorders such as cancers. Various infectious diseases can be diagnosed by the presence of a small quantity of DNA in a clinical specimen specific to an organism, such as a bacterium, yeast, protozoan or virus.

In a preferred embodiment, a method for the detection of a nucleic acid having two complementary strands comprises:

A. contacting the denatured complementary strands of a nucleic acid with:

deoxyribonucleoside-5′-triphosphates,

a DNA polymerase isolated from Thermus aquaticus , Thermus thermophilus or a clone from a genome thereof, and

a pair of primers which are substantially complementary to the denatured nucleic acid strands under hybridizing conditions so as to form hybridized extension products of the primers and their complementary nucleic acid strands, which extension products, when separated from their complements, can serve as templates for synthesis of the extension products,

the primers being present in a molar ratio of at least 5:1,

B. separating the primer extension products from the templates on which they were synthesized,

C. subjecting the separated primer extension products to amplifying conditions for at least 15 cycles, and

D. without further denaturation of the amplified products formed in step C, colorimetrically detecting the presence of the primer extension product which was formed using the primer present in excess,

the detection accomplished using:

a peroxidase-labeled reagent, which in the presence of a peroxidase-responsive leuco dye and hydrogen peroxide, will provide a dye, the reagent capable of being associated with the detected primer extension product, and

a capture probe complementary to the detected primer extension product.

The following examples illustrate the practice of this invention, but are not meant to be limiting in any way.

Examples 1 and 2: HLA Detection Using an Enzyme Label Without a Final Denaturation Step

These examples demonstrate the method of this invention using an enzyme-labeled reagent, a capture probe and colorimetric detection and two different unequal primer ratios. There was no final denaturation step after amplification in this method.

Materials:

A DNA sample was isolated from the HLA homozygous lymphoblastoid cell line LBF (GM 03163 from the Human Genetic Mutant Cell Depository, Camden, N.J.) by proteinase K digestion and phenol/chloroform extraction using the standard procedures described by Maniatis et al (noted above).

The primers used had the following nucleotide sequence using the standard A (adenine), G (guanine), T (thymine) and C (cytosine) designations:

Primer 1 [complementary to the (-) strand]:

5′-X-GTGCTGCAGGTGTAAACTTGTACCAG-3′,

Primer 2 [complementary to the (+) strand]:

5′-X-CACGGATCCGGTAGCAGCGGTAGAGTTG-3′

wherein X represents biotin attached through an aminotetraethylene glycol spacer group using the procedures described in WO-A-0 89/02931.

The capture probe was composed of a polymeric particle formed of poly(styrene-co -acrylic acid) (70:30 molar ratio, 0.7 μmeter average diameter) to which an oligonucleotide complementary to the (-) strand was covalently attached. The oligonucleotide had the following sequence:

5′-Y-CCTCTGTTCCACAGACTTAGATTTG-3′

wherein Y represents an aminotetraethylene glycol spacer group prepared by the procedures described in WO-A-0 89/02931 (noted above).

The polymeric particles were prepared using standard emulsion polymerization methods and the oligonucleotide was attached to the particles using an aminotetraethylene glycol spacer group and standard chemistry using a carbodiimide reagent, such as 3-(dimethylamino)propylethyl carbodiimide hydrochloride.

A Surecell™ disposable test device was used. It contained a 1.2 μmeter pore sized Biodyne™ A nylon microporous membrane which had been coated with casein (about 1 g/m²).

A dye-providing composition included 2-(4-hydroxy-3-methoxyphenyl)-4,5-bis(4-methoxyphenyl)-imidazole leuco dye (0.008 weight %), poly(vinyl pyrrolidone) (1 weight %), sodium phosphate (10 mmolar, pH 6.8), diethylenetriaminepentaacetic acid (10 μmolar), 4′-hydroxyacetanilide (2 mmolar) and hydrogen peroxide (10 mmolar).

Assay:

The DNA from the LBF cell line was amplified in a 100 μliter reaction vessel containing tris-(hydroxymethyl)aminomethane hydrochloride buffer (5 mmolar, pH 8.3), magnesium chloride (2.5 mmolar), each dNTP (175 μmolar each), DNA polymerase isolated from Thermus aquaticus (2 International Units), the primer complementary to the (+) strand (0.2 μmolar) and the primer corresponding to the (-) strand in lesser amounts (either 0.02 or 0.04 μmolar).

The amplification procedure was carried out for 30 cycles (each cycle comprising denaturation for 30 seconds at 95° C, hybridization for 30 seconds at 55° C and primer extension for 60 seconds at 70° C) using a commercially available Perkin-Elmer Thermal cycler. The resulting double-stranded DNA product from the primer in excess was not denatured with a final heating step.

A Control amplification was carried out using the same reagents and procedure except the primers were used in equal amounts and a final denaturation step was carried out at 95° C for five minutes.

Detection of amplified products was accomplished by mixing the polymerase chain reaction mixture described above (100 μl) with the capture probe (200 μg) in a solution containing sodium phosphate (8.5 mmolar, pH 7.4), sodium chloride (149 mmolar), ethylenediaminetetraacetic acid (1 mmolar) and Triton™ X-100 nonionic surfactant (0.1 weight %) to a final volume of 30 μl. This mixture was incubated at 42° C for ten minutes to allow the probe to hybridize to the excess (-) strands. The resulting suspension was added to a Surecell™ disposable test device and the insolubilized product was collected on the membrane in a test well of the test device. The insolubilized product was washed with a solution (200 μl) of sodium phosphate (8.5 mmolar, pH 7.4), sodium chloride (149 mmolar), ethylenediaminetetraacetic acid (1 mmolar) and sodium dodecyl sulfate (0.5 weight %) which had been prewarmed to 55° C.

A horseradish peroxidase-avidin conjugate (2.3 ng) (available in the Kodak See-Quence™ HLA-DQα gene probe kit) was then added to the test device for reaction with the insolubilized biotinylated primer extension product. The test device was incubated at about 20-25° C for two minutes, followed by washing with a solution (200 μl) of tris(hydroxymethyl)aminomethane hydrochloride buffer (50 mmolar, pH 8.8), sodium dodecyl sulfate (2.4 weight %), sodium chloride (0.5 molar) and 1-methyl-2-pyrrolidone (10 weight %). The dye-providing composition (50 μl) was then added followed by incubation at 20-25° C for five minutes. The results of the assays (both Control and invention) are shown in the following Table as a visual dye reading using a scale of 0 to 5 (5 being highest dye density).

While the Control assay provided an acceptable visual signal, this was possible only because the enzyme-labeled reagent was added to the collected product after the denaturation step. This is a limitation for certain assays. It is clear that the present invention provides a means for using an enzyme-labeled reagent to obtain a satisfactory analytical result without the need for a final denaturation after amplification, which heating step would destroy the effectiveness of the enzyme label. It thus provides more flexibility for assays using enzyme-labeled reagents.

TABLE

| Assay | Primer Ratio (Molar) | Dye Density |
|---|---|---|
| Control | 1:1 | 4.5 |
| Example 1 | 5:1 | 4.5 |
| Example 2 | 10:1 | 4.5 |

## Claims

1. A method for the detection of a nucleic acid having two complementary strands comprises:
A. contacting the denatured complementary strands of a nucleic acid with:
deoxyribonucleoside-5′-triphosphates,
an agent for polymerization, and
a pair of primers which are substantially complementary to the denatured nucleic acid strands under hybridizing conditions so as to form hybridized extension products of the primers and their complementary nucleic acid strands, which extension products, when separated from their complements, can serve as templates for synthesis of the extension products,
the primers being present in a molar ratio of at least 2:1,
B. separating the primer extension products from the templates on which they were synthesized,
C. subjecting the separated primer extension products to amplifying conditions, and
D. without further denaturation of the amplified products formed in step C, colorimetrically or fluorometrically detecting the presence of the primer extension product which was formed using the primer in excess, the detection accomplished using an enzyme-labeled reagent, which in the presence of its substrate, will provide a detectable colorimetric or fluorometric signal, the reagent being either complementary to the detected primer extension product, or capable of being associated therewith.

2. The method as claimed in claim 1 wherein the primers are present in a molar ratio of at least 5:1.

3. The method as claimed in either of claims 1 and 2 wherein the agent for polymerization is a thermostable polymerase.

4. The method as claimed in any of claims 1 to 3 wherein the amplified products are detected colorimetrically using a substrate composition for the enzyme-labeled reagent which provides a dye in the presence of the enzyme.

5. The method as claimed in any of claims 1 to 4 wherein the enzyme label is peroxidase and the substrate composition includes a leuco dye which provides a dye in the presence of peroxidase and hydrogen peroxide.

6. The method as claimed in any of claims 1 to 4 wherein the enzyme label is alkaline phosphatase.

7. The method as claimed in any of claims 1 to 6 wherein the enzyme-labeled reagent is an enzyme-labeled oligonucleotide probe complementary to the detected primer extension product.

8. The method as claimed in claim 7 wherein the enzyme label is peroxidase.

9. The method as claimed in any of claims 1 to 8 wherein detection of the primer extension product present in excess includes hybridizing the product with a capture probe.

10. The method as claimed in any of claims 1 to 9 wherein the enzyme-labeled reagent is a conjugate of avidin with an enzyme which is capable of complexing with a biotinylated primer extension product present in excess.

## SEQUENCE LISTING

SEQ ID NO: 1
SEQUENCE TYPE: Nucleotide
SEQUENCE LENGTH: 26 base pairs
STRANDEDNESS: Single
TOPOLOGY: Linear
MOLECULE TYPE: Nucleic acid primer
IMMEDIATE SOURCE: Synthetically prepared
PROPERTIES: Complementary to a nucleic
acid sequence in the (−) strand of
human HLA DNA isolated from the HLA
homozygous lymphoblastoid cell line LBF.
FEATURES: Biotin attached through an
aminotetraethylene glycol spacer group
at 5' position.

GTGCTGCAGG TGTAAACTTG TACCAG

SEQ ID NO: 2
SEQUENCE TYPE: Nucleotide
SEQUENCE LENGTH: 28 base pairs
STRANDEDNESS: Single
TOPOLOGY: Linear
MOLECULE TYPE: Nucleic acid primer
IMMEDIATE SOURCE: Synthetically prepared
PROPERTIES: Complementary to a nucleic
acid sequence in the (+) strand of
human HLA DNA isolated from the HLA
homozygous lymphoblastoid cell line LBF.
FEATURES: Biotin attached through an
aminotetraethylene glycol spacer group
at 5' position.

CACGGATCCG GTAGCAGCGG TAGAGTTG

SEQ ID NO: 3

SEQUENCE TYPE: Nucleotide

SEQUENCE LENGTH: 25 base pairs

STRANDEDNESS: Single

TOPOLOGY: Linear

MOLECULE TYPE: Nucleic acid primer

IMMEDIATE SOURCE: Synthetically prepared

PROPERTIES: Complementary to a nucleic
acid sequence in the (−) strand of
human HLA DNA isolated from the HLA
homozygous lymphoblastoid cell line LBF.
FEATURES: Nucleotide is attached to
polymeric particles through an
aminotetraethylene glycol spacer group
at 5' position.

CCTCTGTTCC ACAGACTTAG ATTTG